# FASCICULE DE BREVET EUROPEEN

(11) **EP 3 599 851 B1**
(45) Date de publication et mention de la délivrance du brevet: **07.10.2026**
(21) Numéro de dépôt: 18711363.4
(22) Date de dépôt: 21.03.2018
(51) Int. Cl.: A01N 25/18, A01N 65/00, A23B 7/144, B65D 81/28

(54) **PROCÉDÉ DE TRAITEMENT PAR AU MOINS UN PRODUIT BIOCIDE ET/OU PHYTOPROTECTEUR MOYENNEMENT VOLATILE, ENSEMBLE DE TRAITEMENT ET ENSEMBLE DE STOCKAGE CORRESPONDANTS**
VERFAHREN ZUR BEHANDLUNG MIT MINDESTENS EINEM LEICHT FLÜCHTIGEN BIOZID UND/ODER PFLANZENSCHUTZMITTEL, ENTSPRECHENDE BEHANDLUNGSANORDNUNG UND LAGERANORDNUNG
PROCESS FOR TREATMENT BY AT LEAST ONE MODERATELY VOLATILE BIOCIDAL AND/OR PLANT-PROTECTION PRODUCT, CORRESPONDING TREATMENT ASSEMBLY AND STORAGE ASSEMBLY

(30) Priorité: 22.03.2017 FR 1752368
(43) Date de publication de la demande: 05.02.2020
(73) Titulaire: Xeda International S.A., 13670 Saint Andiol (FR)
(72) Inventeur: SARDO, Alberto, 13160 Chateaurenard (FR); SARDO, Stefano, 13160 Chateaurenard (FR); PAITEL, Laura, 84130 Le Pontet (FR)
(74) Mandataire: Lavoix
(86) Numéro de dépôt international: PCT/EP2018/057143
(87) Numéro de publication internationale: WO 2018/172402

(56) Documents cités:
- EP-A1- 0 452 512
- US-A1- 2008 251 215
- US-A1- 2016 030 615

## Description

La présente invention concerne les traitements par des produits biocides et/ou phytoprotecteurs moyennement volatiles, notamment pour améliorer la conservation de produits végétaux tels que des céréales ou pour la désinfection des locaux.

Plus précisément, l'invention concerne selon un premier aspect un procédé de traitement par au moins un produit biocide et/ou phytoprotecteur moyennement volatile.

Il est connu que certains produits volatiles de synthèses ou d'origine naturelle, par exemple les huiles essentielles, ont des propriétés biocides ou phytoprotectrices intéressantes. Ils sont notamment utilisés pour traiter des produits végétaux, de manière à améliorer leur conservation.

Les techniques utilisées jusqu'à présent pour appliquer ces produits sur des végétaux sont les suivantes :
- immersion des produits végétaux dans une dispersion aqueuse chaude ou froide contenant le produit ;
- aspersion de locaux, par exemple des chambres de stockage pour végétaux, afin de les assainir ;
- micro-nébulisation à l'entrée des produits végétaux dans les chambres de stockage.

Les quantités de produits appliquées sont élevées et peuvent conduire à des concentrations importantes dans les produits végétaux.

Par ailleurs, les procédés de stérilisation des grains utilisés actuellement sont basés sur un traitement de pulvérisation au moment de l'ensilage de produits hautement toxiques ; des organophosphorés en général. En cas d'attaque d'insectes importante pendant le stockage, le grain est traité ensuite par une produit gazeux ou un produit volatil gazéifié. Les produits utilisés couramment pour cette stérilisation sont la phosphine, le bromure de méthyl ou encore le formiate d'éthyl. Les produits du traitement à l'entrée comme du traitement pendant le stockage sont très toxiques et parfois peu efficaces surtout à cause des problèmes de résistances.

Il y a donc un besoin pour un procédé de traitement qui serait utilisable pour l'application de produits moyennement volatiles d'origine naturelle, par exemple les huiles essentielles, ou synthétiques avec les même caractéristiques de volatilité et qui aurait une excellente efficacité pour la protection des produits végétaux et/ou l'assainissement des locaux, notamment de locaux destinés aux stockages des produits végétaux.

A cette fin, l'invention porte sur un procédé de traitement par au moins un produit biocide et/ou phytoprotecteur moyennement volatile, de température d'ébullition comprise entre 150 et 280°C, le procédé comprenant une étape de traitement au cours de laquelle un liquide contenant un produit ou un mélange de produits biocides et/ou phytoprotecteurs est évaporé et injecté dans l'atmosphère interne d'un local, le liquide étant évaporé à une température inférieure à 50°C, la concentration en vapeur de produit dans l'atmosphère interne du local étant maintenue supérieure à 10% d'une concentration de saturation de la vapeur dudit produit dans ladite atmosphère à ladite température pendant une durée de saturation supérieure à 12 heures, dans lequel le local (2) présente un volume interne supérieur à 200 m³ ; dans lequel le liquide est stocké dans une réserve (7) et est évaporé dans un dispositif d'évaporation (8) comprenant un évaporateur (10) à garnissage (11) par mise en contact avec un flux d'air dans ledit garnissage (11), le flux d'air contenant les vapeurs de produit étant injecté dans le local (2), ledit dispositif d'évaporation (8) comprenant en outre: - un dispositif (12) d'injection d'un flux du liquide dans l'évaporateur à garnissage (10) ; - un organe (13) de circulation du flux d'air dans l'évaporateur à garnissage (10), la réserve (7) étant un bac placé verticalement sous le garnissage (11), le dispositif d'injection (12) étant agencé pour injecter le liquide au-dessus du garnissage (11).

En effet, la demanderesse est convaincue que seule les vapeurs des produits biocides et/ou phytoprotecteurs moyennement volatiles ont une activité, notamment une activité fongicide, bactéricide et insecticide. Les liquides contenant des produits biocides et/ou phytoprotecteurs moyennement volatiles ne sont actifs que comme source de vapeurs.

En conséquence, l'effet biocide et/ou phytoprotecteur est maximum quand on contrôle la concentration de vapeur à une valeur élevée dans l'atmosphère du local à traiter.

Afin d'obtenir les meilleurs résultats, il est indispensable que ces produits soient utilisés sous forme vapeur. Seule la vapeur est immédiatement active et aura les capacités de traiter le volume d'air, de pénétrer dans la masse des produits végétaux stockés (grains en silos, fruits en palox, pommes de terre en tas) et de traiter en profondeur les parois des locaux.

L'utilisation de la vaporisation à chaud pour atteindre des concentrations importantes de vapeurs dans l'atmosphère est fortement déconseillée. En effet, soit on atteint pas la concentration de saturation, soit on la dépasse et il se produit une recondensation des vapeurs en liquide quand la vapeur se refroidit après injection dans l'atmosphère interne du local, notamment sur les surfaces froides. Pour être sur d'avoir une concentration de vapeur élevée, il faudrait donc injecter des quantités élevées de produits, ce qui peut conduire à des concentrations importantes sur les produits végétaux, qui rendrait ces produits phytotoxiques, en produisant en même temps une consommation de produit biocide et/ou phytoprotecteur volatile beaucoup plus grande qu'avec l'évaporation à froid.

La thermonébulisation pose des problèmes semblables. Le produit est injecté à l'état liquide, sous la forme d'un brouillard de gouttelettes. Une partie du produit est perdu, car il se dépose sur les surfaces du local. Une autre partie se dépose sous forme liquide sur les produits végétaux. Le produit n'est pas efficace sous cette forme. Seule la fraction du produit qui se transforme en vapeur est efficace, mais il est extrêmement difficile de contrôler la concentration de vapeur de produit. De nouveau, la consommation de produit biocide et/ou phytoprotecteur volatile est beaucoup plus grande qu'avec l'évaporation à froid.

Avec le procédé de l'invention, l'évaporation est réalisée à froid, ce qui permet un excellent contrôle de la concentration de vapeur de produit dans l'atmosphère interne du local. Cette concentration peut être maintenue élevée, proche de la saturation si nécessaire, pour maximiser l'effet du produit. La consommation de produit biocide et/ou phytoprotecteur volatile est modérée comparée à la thermonébulisation ou à la vaporisation à chaud, car la quantité totale du biocide exerce son activité.

La consommation de produit est autorégulée. Le produit biocide et/ou phytoprotecteur étant vaporisé à température ambiante, inférieure à 50°C, il s'évapore jusqu'à atteindre au maximum la saturation de l'atmosphère sans risque de sursaturation. Une fois ce maximum atteint, l'évaporation s'arrête naturellement, sans action extérieure, même si le dispositif d'évaporation continu à fonctionner.

L'efficacité du traitement provient également du fait que la concentration de la vapeur de produit dans l'atmosphère est maintenue à un niveau élevée pendant une durée dite de saturation significative, cette durée de saturation étant supérieure à 12 heures. Le traitement peut s'effectuer soit en injectant rapidement une quantité importante de produit, soit en répartissant les injections de manière à maintenir la concentration élevée pendant une durée suffisante pour garantir une efficacité.

Trois modes d'application préférés ont ainsi été envisagés, basés soit sur le principe d'un effet choc, avec une concentration de vapeur de produit proche de la concentration de saturation, pour une période courte, soit avec une concentration plus faible pour des périodes longues. En effet l'efficacité de ces produits dépend de la concentration dans l'air et du temps de contact avec le produit végétal.

Le premier mode d'application est par injection unique, la concentration de vapeur du ou de chaque produit dans l'atmosphère interne du local étant maintenue entre 50 et 100% de la concentration de saturation pour une durée comprise entre 12 et 240 heures afin d'obtenir un effet de stérilisation complète des produits végétaux.

Le deuxième mode d'application est par le maintien pendant la plus grande partie voire sensiblement toute la période de stockage d'une concentration moins importante de vapeur du ou de chaque produit dans l'atmosphère interne du local, comprise entre 10 et 50% de la concentration de saturation.

Selon un troisième mode d'application, des injections du même type que dans le premier mode d'application sont effectuées, de manière périodique, soit quand l'augmentation du risque a été constaté visuellement, soit selon un programme prédéterminé, par exemple 5 jours d'injection tous les mois de stockage.

Pour ces trois modes d'application, le contrôle de la quantité injectée peut se faire de différentes façons.

Selon une première méthode, le contrôle de la quantité injectée se fait en laissant l'évaporation s'arrêter naturellement, quand la saturation de l'atmosphère interne du local est atteinte. La quantité de liquide évaporée dépend des quantités d'air et de liquide mises en contact et de la surface de contact du dispositif d'évaporation.

Selon une seconde méthode, le contrôle de la quantité injectée se fait en suivant un programme de fonctionnement prédéterminé, indiquant la durée de fonctionnement par jour et la quantité de liquide évaporée par unité de volume du local pendant ladite durée de fonctionnement.

Selon une troisième méthode, la concentration du ou de chaque produit dans l'air est analysée, le dispositif d'injection étant piloté en utilisant le résultat de l'analyse, typiquement pour maintenir la concentration du ou de chaque produit dans une fourchette prédéterminée.

Le procédé de traitement peut également présenter une ou plusieurs des caractéristiques ci-dessous, considérées individuellement ou selon toutes les combinaisons techniquement possibles :
- au moins un des produits est choisi dans la liste de produits phytoprotecteurs ou biocides suivantes :
   huile essentielle ; terpène ; alcool à chaîne courte de C6 à C10 saturé ou insaturé, comme par exemple, l'octanol, le 2-éthylhexanol ; produit de synthèse volatile, comme par exemple, l'hexanal, le diméthylnaphtalène et le 3-décène-2-one ; les acides organiques liquides à point d'ébullition élevé, tels que l'acide pélargonique et l'acide parabénique ; les esters avec activité biocide comme par exemple l'isovalérianate d'isoamyle ;
- le local contient des produits végétaux, par exemple des céréales, stockés pendant une durée de stockage (DS), normalement supérieure à un mois ;
- l'étape de traitement comprend une phase d'injection continue de durée supérieure à 50% de la durée de stockage (DS), de préférence supérieure à 75% de la durée de stockage (DS) et encore de préférence supérieure à 90% de la durée de stockage ;
- l'étape de traitement comprend une seule phase d'injection continue ayant une durée d'injection de 12 à 240 heures, ou comprend plusieurs phases d'injection continues séparées par des phases d'attente sans injection de produit biocide et/ou phytoprotecteur, chaque phase d'injection continue ayant une durée d'injection (DI) entre 12 heures et 240 heures ;
- le liquide comprend plusieurs produits biocides et/ou phytoprotecteurs moyennement volatiles, ayant des pressions de vapeur respectives différentes les unes des autres à la température ambiante ;
- les pressions de vapeur respectives des produits biocides et/ou phytoprotecteurs volatiles s'étalent sur un intervalle de pression entre 1,3.10⁻⁵ bar et 4.10⁻³ bar ;
- l'atmosphère interne du local est chauffée pendant l'injection de la vapeur de produit, à une température comprise entre la température normale du local et la température normale plus 5°C ;
- le local est un silo à grains ayant une entrée d'air communiquant avec l'extérieur et une sortie d'air communiquant avec l'extérieur, le silo comprenant une ventilation forcée prévue pour assurer une circulation d'air de l'entrée d'air à la sortie d'air à travers les grains, la ventilation forcée étant à l'arrêt pendant l'injection du ou de chaque produit biocide et/ou phytoprotecteur ;
- le local présente un volume interne supérieur à 200 m³ ;
- le liquide est évaporé par mise en contact avec un flux d'air dans un garnissage, le flux d'air contenant les vapeurs de produit étant injecté dans le local ;
- le local est sensiblement étanche à l'air, le flux d'air circulant dans le garnissage ayant un débit compris entre 1 et 10 m³ par heure et par m³ de volume interne du local ;
- le flux d'air circulant dans le garnissage a un débit compris entre 1 et 6 m3 par heure et par 100 m³ de volume interne du local.

Selon un second aspect, l'invention porte sur un ensemble de traitement, l'ensemble comprenant :
- une réserve de liquide contenant au moins un produit ou un mélange de produits biocides et/ou phytoprotecteurs moyennement volatiles, de température d'ébullition comprise entre 150 et 280°C ;
- un dispositif d'évaporation du liquide stocké dans la réserve et d'injection du liquide évaporé dans l'atmosphère interne d'un local, le dispositif d'évaporation étant configuré pour évaporer le liquide à une température inférieure à 50°C,
- un contrôleur contrôlant le dispositif d'évaporation, configuré pour que la concentration en vapeur du ou de chaque produit dans l'atmosphère interne du local soit maintenue supérieure à 10% d'une concentration de saturation de la vapeur dudit produit dans ladite atmosphère pendant une durée de saturation supérieure à 12 heures, s ; dans lequel le local (2) présente un volume interne supérieur à 200 m³ ; dans lequel le dispositif d'évaporation (8) comprend un évaporateur (10) à garnissage dans lequel le liquide est évaporé par mise en contact avec un flux d'air dans ledit garnissage (11), l'évaporateur (10) étant configuré pour injecter le flux d'air contenant les vapeurs de produit dans le local (2), ledit dispositif d'évaporation (8) comprenant en outre: - un dispositif (12) d'injection d'un flux du liquide dans l'évaporateur à garnissage (10) ; - un organe (13) de circulation du flux d'air dans l'évaporateur à garnissage (10) ; 22 la réserve (7) étant un bac placé verticalement sous le garnissage (11), le dispositif d'injection (12) étant agencé pour injecter le liquide au-dessus du garnissage (11).

L'ensemble de traitement peut également présenter une ou plusieurs des caractéristiques ci-dessous, considérées individuellement ou selon toutes les combinaisons techniquement possibles :
- au moins un des produits est choisi dans la liste de produits phytoprotecteurs ou biocides suivante :
   huile essentielle ; terpène ; alcool à chaîne courte de C6 à C10 saturé ou insaturé, comme par exemple, l'octanol, le 2-éthylhexanol ; produit de synthèse volatile, comme par exemple le l'hexanal, le diméthylnaphtalène et le 3-décène-2-one ; les acides organiques liquides à point d'ébullition élevé, tels que l'acide pélargonique et l'acide parabénique, les esters avec activité biocide comme par exemple l'isovalérianate d'isoamyle ;
- le local contient des produits végétaux, par exemple des pommes de terre, stockés pendant une durée de stockage supérieure à un mois, le contrôleur étant configuré pour réaliser une phase d'injection continue ayant une durée d'injection supérieure à 50% de la durée de stockage, de préférence supérieure à 75% de la durée de stockage et encore de préférence supérieure à 90% de la durée de stockage ;
- le local contient des produits végétaux, par exemple des céréales, le contrôleur étant configuré pour réaliser une seule phase d'injection continue ayant une durée d'injection de 12 à 240 heures, ou plusieurs phases d'injection continues séparées par des phases d'attente sans injection de produit biocide et/ou phytoprotecteur, chaque phase d'injection continue ayant une durée d'injection de 12 à 240 heures ;
- le liquide comprend plusieurs produits biocides et/ou phytoprotecteurs moyennement volatiles, ayant des pressions de vapeur respectives différentes les unes des autres ;
- les pressions de vapeur respectives des produits biocides et/ou phytoprotecteurs moyennement volatiles s'étalent sur un intervalle de pression entre 1,3.10⁻⁵ bar et 4.10⁻³ bar ;
- l'ensemble de traitement comprend un chauffage de l'atmosphère interne du local, configuré pour chauffer ladite atmosphère interne pendant l'injection de la vapeur de produit, à une température comprise entre la température normale du local et la température normale plus 5°C ;
- le dispositif d'évaporation comprend un évaporateur à garnissage dans lequel le liquide est évaporé par mise en contact avec un flux d'air dans ledit garnissage, l'évaporateur étant configuré pour injecter le flux d'air contenant les vapeurs de produit dans le local.

Selon un troisième aspect, l'invention porte sur un ensemble de stockage de produits végétaux, l'ensemble de stockage comprenant :
- un local, contenant de préférence des produits végétaux ; et
- un ensemble de traitement comme dans la revendication 7, configuré pour injecter le liquide évaporé dans l'atmosphère interne du local.

L'ensemble de stockage peut également présenter une ou plusieurs des caractéristiques ci-dessous, considérées individuellement ou selon toutes les combinaisons techniquement possibles :
- le local présente un volume interne supérieur à 200 m³ ;
- le local est sensiblement étanche à l'air, l'ensemble de traitement comprenant un évaporateur à garnissage dans lequel le liquide est évaporé par mise en contact avec un flux d'air dans ledit garnissage, l'évaporateur étant configuré pour injecter le flux d'air contenant les vapeurs de produit dans le local, le contrôleur étant programmé pour que le flux d'air circulant dans le garnissage ait un débit compris entre 1 et 10 m³ par heure et par m³ de volume interne du local ;
le local est un silo à grains ayant une entrée d'air communiquant avec l'extérieur et une sortie d'air communiquant avec l'extérieur, le silo comprenant une ventilation forcée prévue pour assurer une circulation d'air de l'entrée d'air à la sortie d'air à travers les grains, la ventilation forcée étant susceptible d'être mise à l'arrêt pendant l'injection du ou de chaque produit biocide et/ou phytoprotecteur, le contrôleur étant programmé pour que le flux d'air circulant dans le garnissage ait un débit compris entre 1 et 6 m³ par heure et par 100 m³ de volume interne du local.

D'autres caractéristiques et avantages de l'invention ressortiront de la description ci-dessous, donnée à titre indicatif et nullement limitatif, en référence aux figures annexées, parmi lesquelles :
- la figure 1 est une représentation schématique simplifiée d'un ensemble de stockage conforme à un premier mode de réalisation de l'invention ; et
- la figure 2 est une représentation schématique simplifiée d'un ensemble de stockage conforme à un second mode de réalisation de l'invention.

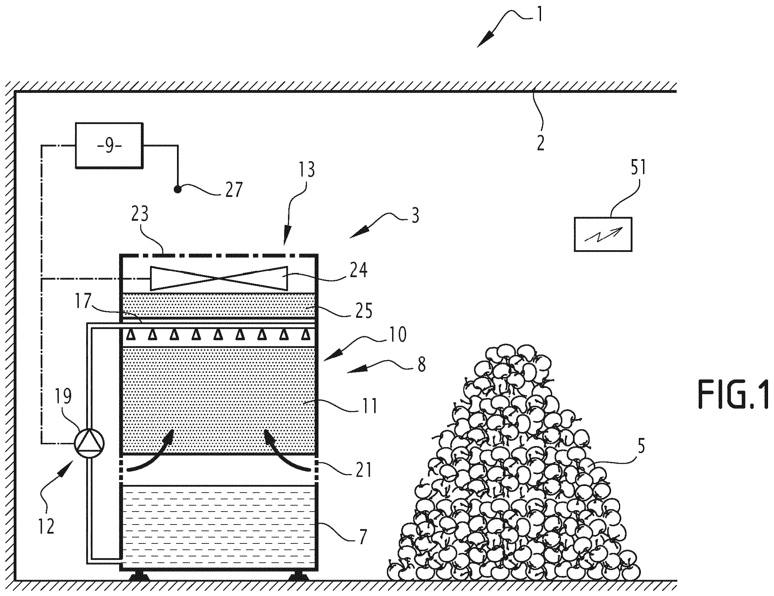

L'ensemble de stockage 1 illustré sur la figure 1 comprend un local 2 et un ensemble de traitement 3.

L'ensemble de traitement 3 est configuré pour évaporer un liquide contenant au moins un produit biocide et/ou phytoprotecteur moyennement volatile et pour injecter le liquide évaporé dans l'atmosphère interne du local 2.

Le traitement est un traitement de désinfection et/ou un traitement phytoprotecteur.

Dans le premier mode de réalisation, le local est sensiblement étanche à l'air. Le local est une enceinte fermée, au sens où les échanges entre l'atmosphère du local et l'extérieur, notamment les échanges gazeux, sont réduits, de manière par exemple à ne pas mettre en péril la conservation de produits végétaux qui y seraient stockés.

Dans le cas d'un traitement de désinfection, le local est par exemple un stockage destiné de produits végétaux, mais ne contenant pas de produits végétaux au moment du traitement. En variante, le local est une partie d'un hôpital, d'une école, d'une installation industrielle, ou tout autre type de local. Le local peut encore être une cuve, une citerne de stockage ou de transport, ou n'importe quel autre type d'enceinte à désinfecter.

Dans le cas d'un traitement phytoprotecteur, le local est par exemple une chambre, une serre ou tout local destiné au stockage de produits végétaux tels que des grains de céréales, des fruits ou des légumes. Un tel local est représenté sur la figure 1. Le traitement est appliqué pendant que les produits végétaux 5 sont stockés dans le local. En variante, il est appliqué pendant que le local est vide.

L'ensemble de traitement 3 est spécialement destiné au traitement de l'atmosphère d'un local de grand volume. Le volume du local est typiquement supérieur à 200 m³, de préférence supérieur à 500 m³ et encore de préférence supérieur à 1000 m³.

L'ensemble de traitement 3 comprend :
- une réserve 7 stockant un liquide contenant au moins un produit biocide et/ou phytoprotecteur moyennement volatile, de température d'ébullition comprise entre 150 et 280°C, de préférence entre 150 et 260°C ;
- un dispositif 8 d'évaporation du liquide stocké dans la réserve 7 et d'injection du liquide évaporé dans l'atmosphère interne du local 2, le dispositif d'évaporation 8 étant configuré pour évaporer le liquide à une température inférieure à 50°C,
- un contrôleur 9 contrôlant le dispositif d'évaporation 8.

Le dispositif d'évaporation 8 comprend de préférence un évaporateur 10 à garnissage 11 dans lequel le liquide est évaporé par mise en contact avec un flux d'air dans ledit garnissage 11.

L'évaporateur 10 est configuré pour injecter le flux d'air contenant les vapeurs de produit dans le local 2.

Le dispositif d'évaporation 8 comprend en outre:
- un dispositif 12 d'injection d'un flux du liquide dans l'évaporateur à garnissage 10 ;
- un organe 13 de circulation du flux d'air dans l'évaporateur à garnissage 10.

L'évaporateur est de tout type adapté. Par exemple, l'évaporateur est une tour à garnissage, d'axe vertical dans l'exemple représenté.

On entend ici par garnissage tout type de structure permettant d'obtenir une surface de contact importante entre une phase liquide et une phase gazeuse, et ainsi d'améliorer les échanges entre la phase liquide et la phase gazeuse.

Le garnissage peut ainsi être un garnissage de type vrac ou un garnissage de type structuré.

Dans le cas présent, le garnissage est par exemple du type anneau de Raschig ou anneau de Pall, ou encore est un garnissage structuré en nids d'abeilles.

Il est typiquement réalisé dans une matière plastique.

La mise en contact permet de réaliser des transferts particulièrement efficaces entre le flux de liquide et le flux d'air.

Avantageusement, le dispositif d'évaporation est placé dans le local 2. L'organe de circulation 13 aspire directement l'atmosphère interne du local et fait circuler celle-ci dans le garnissage, cette atmosphère constituant donc le flux d'air.

L'atmosphère du stockage correspond ici au volume des gaz remplissant le local et baignant le cas échéant les produits végétaux.

Cette atmosphère comprend typiquement de l'air, plus le cas échéant les gaz et les produits dégagés par les produits végétaux au cours de leur maturation. Elle comprend également de la vapeur d'eau.

En variante, l'atmosphère est une atmosphère modifiée, l'air étant par exemple appauvri en oxygène. Ceci est le cas notamment pour le stockage de certains produits végétaux comme les pommes.

La réserve 7 est typiquement un bac, placé verticalement sous le garnissage 11.

Le dispositif d'injection 12 est agencé pour injecter le liquide au-dessus du garnissage 11.

A cet effet, il comprend typiquement une ou plusieurs rampes d'aspersion 17, placées au-dessus du garnissage, et un organe de transfert 19, tel qu'une pompe, aspirant le liquide dans la réserve 7 et refoulant celui-ci dans la ou les rampes 17.

Le dispositif de circulation 13 est agencé pour créer une circulation ascendante de l'air à l'intérieur de l'évaporateur 10.

Pour ce faire, le dispositif d'évaporation 8 comporte une ou plusieurs entrées 21 pour l'atmosphère débouchant à l'intérieur de l'évaporateur 10, sous le garnissage 11.

Chaque entrée 21 communique fluidiquement avec l'intérieur du local 2.

Le dispositif d'évaporation 8 présente une sortie 23 pour l'atmosphère chargée en liquide évaporé, placée en partie supérieure de l'évaporateur 10, au-dessus des garnissages 11. La sortie 23 est raccordée fluidiquement avec l'intérieur du local 2.

Le dispositif de circulation 13 comprend par exemple un organe de circulation 24 tel qu'un ventilateur ou une soufflante, placé au-dessus du garnissage 11, typiquement au sommet du dispositif d'évaporation 10.

L'organe de circulation 24 aspire l'atmosphère chargée en liquide évaporé au-dessus du garnissage 11, et le refoule dans ou vers la sortie 23.

De préférence, le dispositif d'évaporation 8 comporte un séparateur de gouttes 25, placé au-dessus des rampes d'aspersion 17, et plus précisément entre les rampes d'aspersion 17 et l'organe de circulation 24.

Dans un exemple de réalisation, le dispositif d'évaporation 8 présente une section horizontale carrée, sensiblement constante, de 700x700 mm. La réserve 7 présente la même section horizontale, et présente une hauteur comprise entre 500 et 700 mm. Le dispositif présente quatre entrées 21, chacune disposée sur un des côtés. Le garnissage 11 présente une hauteur d'environ 1 m. Le garnissage est placé par exemple 700 mm au-dessous de l'arrivée de liquide, le séparateur de gouttes 25 étant placé 300 mm au-dessus de l'arrivée de liquide.

L'ensemble de traitement 1 comporte de préférence un capteur 27 de mesure de la concentration du produit biocide et/ou phytoprotecteur moyennement volatile dans l'atmosphère, le contrôleur 9 étant renseigné par le capteur 27.

Le contrôleur 9 est programmé pour piloter le dispositif d'injection 12 et/ou l'organe de circulation 24.

Le contrôleur 9 est par exemple un calculateur ou une partie de calculateur. En variante, le dispositif électronique de pilotage 29 est réalisé sous forme de composants logiques programmables (FPGA, Field Programmable Gate Area) ou sous forme d'un circuit intégré dédié (ASIC, Application Specific Integrated Circuit). Le dispositif électronique 29 est programmé pour mettre en œuvre une stratégie de traitement.

Quand le produit est un produit biocide, le traitement vise à assainir le local.

Quand le produit est un produit phytoprotecteur, qui peut aussi être appelé produit phytosanitaire, le traitement vise à protéger les produits végétaux, en empêchant le développement de maladies et/ou des pourritures, et en éliminant les insectes, champignons et parasites, notamment les champignons à l'origine des mycotoxines.

Le liquide ne contient que des produits biocides, ou que des produits phytoprotecteurs, ou encore comprend un ou plusieurs produits biocides mélangés à un ou plusieurs produits phytoprotecteurs.

Au moins un des produits est choisi dans la liste suivante : huile essentielle ; terpènes ; alcool de C6 à C10 saturé ou insaturé, comme par exemple, l'octanol, le 2-éthylhexanol ; les produits de synthèse volatiles, comme par exemple le l'hexanal, le diméthylnaphtalène et le 3-décène-2-one ; les acides organiques liquides à point d'ébullition élevé, tels que l'acide pélargonique et l'acide parabénique, les esters avec activité biocide comme par exemple l'isovalérianate d'isoamyle.

L'huile essentielle est par exemple choisie dans le groupe formé par l'huile de menthe, l'huile de girofle, l'huile de rose, l'huile de thym, l'huile d'origan, l'huile d'eucalyptus, l'huile de pin, l'huile de cannelle. En variante, le liquide comprend l'un des constituants de ces huiles, choisi dans l'ensemble formé par la L-carvone, l'eugénol, le géraniol, le thymol, le carvacrol, l'eucalyptol, le pinène, le cinnamaldéhyde.

Chaque produit biocide et/ou phytoprotecteur présente typiquement une tension de vapeur comprise entre 0.01 mmHg et 3 mmHg, c'est-à-dire entre 1,3 et 400 Pa.

Typiquement, le liquide comprend seulement le ou les produits, sans solvant ni adjuvant. En variante, le liquide comporte un solvant aqueux ou organique, dans lequel est dissout le ou les produits et un ou plusieurs adjuvants. Le solvant aqueux est par exemple de l'eau. Le solvant organique est par exemple un solvant du type décrit dans FR 2 791 910 ou des glycols, di-glycols et leurs esters relatifs. Les adjuvants sont par exemple des substances aptes à véhiculer la ou les matières actives ou aptes à donner un effet de dilution.

En tout état de cause, le liquide au cours de l'étape de mise en contact est vaporisé à une température inférieure à 50°C, de préférence inférieure à 20°C, notamment comprise entre -2°C et + 12°C, et en particulier entre 0 et 10°C. Par exemple, le liquide est évaporé à température ambiante.

Le contrôleur 9 est configuré pour que la concentration en vapeur de produit dans l'atmosphère interne du local 2 soit maintenue supérieure à 10% d'une concentration de saturation de la vapeur dudit produit dans ladite atmosphère pendant une durée de saturation supérieure à 12 heures.

De préférence, le contrôleur 9 est configuré pour que la concentration en vapeur de produit dans l'atmosphère interne du local 2 soit maintenue supérieure à 30% de la concentration de saturation de la vapeur dudit produit dans ladite atmosphère pendant une durée de saturation supérieure à 12 heures, de préférence maintenue supérieure à 50%, encore de préférence supérieure à 80%, et idéalement supérieure à 90% de la saturation.

La concentration de saturation de chaque produit est fonction du produit et de la température dans le local. Elle correspond à la concentration maximum possible de vapeur de produit dans l'atmosphère, aucune évaporation supplémentaire de produit n'étant possible une fois la saturation atteinte, à température constante.

La durée de saturation est égale à la somme de toutes les périodes pendant lesquelles la concentration reste supérieure à la limite visée, à savoir 10% de la concentration de saturation, ou 30% ou 50% ou 80% ou 90%. Cette durée est continue ou se décompose en plusieurs périodes séparées par des périodes où la concentration de vapeur de produit est inférieure à la limite visée.

De préférence, la durée de saturation est supérieure à 2 jours par mois, encore de préférence supérieure à 5 jours par mois.

Le pilotage peut être réalisé de différentes façons.

Selon une première variante, le contrôleur 9 fait fonctionner le dispositif d'évaporation 8 en continu. La concentration de la vapeur du produit ou du mélange de produits biocide et/ou phytoprotecteur dans le local augmente rapidement jusqu'à atteindre la saturation. L'évaporation s'arrête ensuite naturellement, bien que le dispositif d'évaporation continue à fonctionner. Seules sont évaporées de faibles quantités de produits, compensant l'absorption par les produits végétaux et les fuites vers l'extérieur du local. La concentration de la vapeur du produit ou du mélange de produits biocide et/ou phytoprotecteur est maintenue proche de 100% de la saturation, en permanence.

Selon une seconde variante, le contrôleur 9 est programmé pour régler le débit du flux de liquide en fonction de la concentration mesurée par le capteur 27. De préférence, il pilote également l'organe de circulation 24, de la même manière. Il pilote donc la quantité de produit biocide et/ou phytoprotecteur évaporée pour maintenir la concentration de vapeur de produit mesurée par le capteur 27 conforme à un diagramme de temps préprogrammé, correspondant à la stratégie de traitement souhaitée. Le diagramme de temps indique l'évolution souhaitée pour la concentration de vapeur de produit en fonction du temps.

Selon une troisième variante, le contrôleur 9 est programmé pour régler le débit du flux de liquide en fonction d'un diagramme de temps préprogrammé, correspondant à la stratégie de traitement souhaitée. De préférence, il pilote également l'organe de circulation 24, de la même manière. Le contrôleur 9 pilote donc la quantité de produit biocide et/ou phytoprotecteur évaporée selon le diagramme de temps préprogrammé. Celui-ci indique l'évolution souhaitée pour la quantité de vapeur de produit évaporée en fonction du temps

Plusieurs modes d'applications sont envisagés comme indiqué plus haut.

Selon le premier mode d'application, le contrôleur 9 est configuré pour réaliser une unique phase d'injection de vapeur du ou de chaque produit, une concentration comprise entre 50 et 100% de la concentration de saturation étant maintenue dans l'atmosphère interne du local de manière continue pendant une durée comprise entre 12 et 240 heures afin d'obtenir un effet de stérilisation complète des produits végétaux ou des locaux. La durée est de préférence comprise entre 24 heures et 120 heures, encore de préférence comprise entre 24 heures et 72 heures. La concentration visée est de préférence supérieure à 70%, encore de préférence supérieure à 90% de la saturation.

Selon ce mode d'application, le diagramme des temps comporte un seul créneau, de courte durée.

Selon un second mode d'application, le contrôleur 9 est configuré pour réaliser une seule phase d'injection, continue.

Dans le cas où le local contient des produits végétaux, par exemple des pommes de terre, stockés pendant une durée de stockage DS, la phase d'injection continue a typiquement une durée d'injection DI supérieure à 50% de la durée de stockage DS, de préférence supérieure à 75% de la durée de stockage DS et encore de préférence supérieure à 90% de la durée de stockage DS.

Typiquement la durée de stockage est supérieure à un mois, et est par exemple comprise entre un et 9 mois.

Selon ce mode d'application, le diagramme des temps comporte un seul créneau, continu, de longue durée. La concentration de la vapeur de ou de chaque produit biocide et/ou phytoprotecteur dans l'atmosphère interne du local est maintenue entre 10 et 50% de la saturation, pendant sensiblement toute la durée d'injection.

Selon un troisième mode d'application, le contrôleur 9 est configuré pour réaliser plusieurs phases d'injection continues séparées par des phases d'attente sans injection de produit biocide et/ou phytoprotecteur.

Chaque phase d'injection continue est du type décrit pour le premier mode d'application. Elle a une durée d'injection DI entre 12 heures et 240 heures, de préférence comprise entre un jour et cinq jours. La concentration de la vapeur de ou de chaque produit biocide et/ou phytoprotecteur est donc maintenue dans l'atmosphère interne du local entre 50% et 100% de la saturation à chaque phase d'injection pendant une durée de saturation supérieure à 12 heures, de préférence comprise entre un jour et cinq jours

Les phases d'attente ont par exemple une durée comprise entre 10 jours et 2 mois, de préférence entre 20 jours et 40 jours, et de longueur typiquement égale à 30 jours moins la durée de la phase d'injection.

Les phases d'injection ont typiquement toutes la même durée. De même, les phases d'attente ont typiquement toutes la même durée.

En variante, les phases d'injection et/ou les phases d'attente ont des durées différentes les unes des autres.

Selon cette stratégie, le diagramme des temps comporte plusieurs créneaux successifs.

Les quantités de chaque produit biocide et/ou phytoprotecteur évaporées à chaque phase d'injection, et la durée de chaque phase d'injection, sont choisies pour atteindre rapidement la limite de concentration en vapeur visée, et pour que cette concentration soit maintenue suffisamment longtemps pour obtenir l'effet biocide et/ou phytoprotecteur recherché.

La durée de chaque phase d'attente est choisie suffisamment courte pour que les maladies, pourritures, insectes, champignons ou parasites ne puissent pas se développer de manière significative avant la phase d'injection suivante.

Comme indiqué plus haut, une nouvelle phase d'injection est décidée quand l'augmentation du risque a été constatée visuellement. En variante, les phases d'injection et d'attente sont selon un programme prédéterminé, par exemple 5 jours d'injection tous les mois de stockage, suivis de 25 jours sans injection.

Ainsi, l'invention vise à obtenir rapidement la limite de concentration en vapeur visée. On cherche par ce moyen à parfaitement traiter tout le local et son contenu, en saturant l'atmosphère très rapidement, la vapeur du produit agissant ainsi de façon immédiate et optimale en tous points du local.

Cet effet peut être obtenu du fait que la surface de contact entre le liquide et l'atmosphère est élevée, du fait de la présence des garnissages. L'ensemble de traitement de la présente invention offre une surface de contact entre le gaz et le liquide qui peut aller par exemple jusqu'à 300 m².

Ceci permet d'utiliser des débits d'air et des débits de liquide très important.

Quand le local 2 est sensiblement étanche à l'air, le flux d'air circulant dans le garnissage 11 a un débit compris entre 1 et 10 m³ par heure et par m³ de volume interne du local, de préférence entre 5 et 10 par heure et par m³ de volume interne du local .

Le débit de liquide dans le garnissage 11 est typiquement compris entre 1 et 30 m³/h.

On peut ainsi évaporer des quantités de produits importantes, par exemple 20 litres de produit par jour, et atteindre rapidement la concentration de saturation du produit dans l'atmosphère.

En variante, l'évaporateur à garnissage est remplacé par la machine vendue sous le nom de XEDAVAP, faisant l'objet de la demande de brevet déposé sous le numéro FR1255999. Dans une telle machine, le liquide à évaporer est injecté dans une toile balayée par un courant d'air. La toile a une surface développée comprise entre 1 m² et 4 m². Le débit d'air est compris entre 1000 et 3000 m³/heure. Une telle machine permet d'évaporer entre 0,1 et 10 litres de liquide par jour, par exemple 1,2 litre/jour d'huile de menthe. Une telle machine est prévue pour des locaux plus petits que l'évaporateur à garnissage.

Le fonctionnement de l'ensemble de traitement avec un évaporateur à garnissage est le suivant.

Le liquide à évaporer est disposé dans la réserve 7. L'organe de transfert 19 refoule le liquide dans la ou les rampes 17, qui projettent le liquide vers le garnissage 11. L'organe 24 de mise en circulation de l'atmosphère crée un flux gazeux ascendant. L'atmosphère pénètre dans le dispositif de traitement 8 par les entrées 21, circule vers le haut à travers le garnissage 11. Le liquide circule quant à lui vers le bas à travers le garnissage 11, une partie du liquide étant évaporée au contact du flux gazeux et étant entraînée avec l'atmosphère sous forme de vapeur. La fraction du liquide qui n'est pas évaporée retombe dans la réserve 7. Elle est recyclée. L'atmosphère chargée en liquide évaporé passe à travers le séparateur de gouttes 25 et est refoulée par l'organe de circulation 24 jusqu'à la sortie 23.

L'ensemble de traitement 8 rejette cette atmosphère chargée en vapeur directement dans le stockage, par la sortie 23.

Le débit de liquide est par exemple de 3 m³/heure, et le débit d'air d'environ 2000 m³/heure.

L'invention vise aussi un procédé de traitement par au moins un produit biocide et/ou phytoprotecteur moyennement volatile, de température d'ébullition comprise entre 150 et 280°C.

Le procédé comprend une étape de traitement au cours de laquelle un liquide contenant un produit ou un mélange de produits biocides et/ou phytoprotecteurs est évaporé et injecté dans l'atmosphère interne d'un local 2, le liquide étant évaporé à une température inférieure à 50°C.

La concentration en vapeur de produit dans l'atmosphère interne du local 2 est maintenue supérieure à 10% d'une concentration de saturation de la vapeur dudit produit dans ladite atmosphère pendant une durée de saturation supérieure ou égale à 12 heures.

Le ou les produits sont du type décrit plus haut pour l'ensemble de traitement.

Le local contient typiquement des produits végétaux, par exemple des céréales, stockés pendant une durée de stockage DS supérieure à un mois, ou des plantes dans une serre.

Selon un premier mode d'application, l'étape de traitement comprend une injection unique de vapeur du produit ou du mélange des produits, la concentration du ou de chaque produit dans l'atmosphère interne du local étant maintenue comprise entre 50 et 100% de la concentration de saturation pour une durée comprise entre 12 et 240 heures afin d'obtenir un effet de stérilisation complète des produits végétaux.

Selon un deuxième mode d'application, l'étape de traitement comprend une injection unique, une concentration moins importante de vapeur du produit ou du mélange de produit, comprise entre 10 et 50% de la concentration de saturation, étant maintenue dans l'atmosphère interne du local pendant la plus grande partie voire sensiblement toute la période de stockage.

Selon un troisième mode d'application, l'étape de traitement comprend plusieurs injections du même type que dans le premier mode d'application, réalisées de manière périodique, soit quand l'augmentation du risque a été constaté visuellement, soit selon un programme prédéterminé, par exemple 5 jours d'injection tous les mois de stockage.

Ces modes de traitement sont comme décrit plus haut relativement à l'ensemble de traitement.

Le local présente un volume interne supérieur à 200 m³. Il est du type décrit plus haut relativement à l'ensemble de traitement.

Avantageusement, le liquide est évaporé par mise en contact avec un flux d'air dans un garnissage 11, le flux d'air contenant les vapeurs de produit étant injecté dans le local 2.

Le liquide est de préférence évaporé dans un dispositif d'évaporation du type décrit plus haut relativement à l'ensemble de traitement.

Dans le premier mode de réalisation, le local 2 est sensiblement étanche à l'air, le flux d'air circulant dans le garnissage ayant un débit compris entre 1 et 10 m³ par heure et par m³ de volume interne du local.

Selon une première variante, le dispositif utilisé pour évaporer le liquide fonctionne en continu. La concentration de la vapeur de ou de chaque produit biocide et/ou phytoprotecteur dans le local augmente rapidement jusqu'à atteindre la saturation. L'évaporation s'arrête ensuite naturellement, bien que le dispositif d'évaporation continue à fonctionner. La concentration de la vapeur de ou de chaque produit biocide et/ou phytoprotecteur est maintenue proche de 100% de la saturation, en permanence.

Selon une deuxième variante, la quantité de produit biocide et/ou phytoprotecteur évaporée suit un diagramme de temps préprogrammé, correspondant à la stratégie de traitement recherchée. Celui-ci indique l'évolution souhaitée pour la quantité de vapeur de produit évaporée en fonction du temps

Selon une troisième variante, la concentration en vapeur du ou de chaque produit dans l'atmosphère du local est mesurée en permanence. La quantité de produit biocide et/ou phytoprotecteur évaporée est choisie pour maintenir la concentration de vapeur de produit mesurée par le capteur 27 conforme à un diagramme de temps préprogrammé, correspondant à la stratégie de traitement souhaitée. Le diagramme de temps indique l'évolution souhaitée pour la concentration de vapeur de produit en fonction du temps.

Le procédé est prévu pour être mis en œuvre par l'ensemble de traitement 8 décrit ci-dessus. Inversement, l'ensemble de traitement 8 décrit ci-dessus est particulièrement adapté pour la mise en œuvre du procédé.

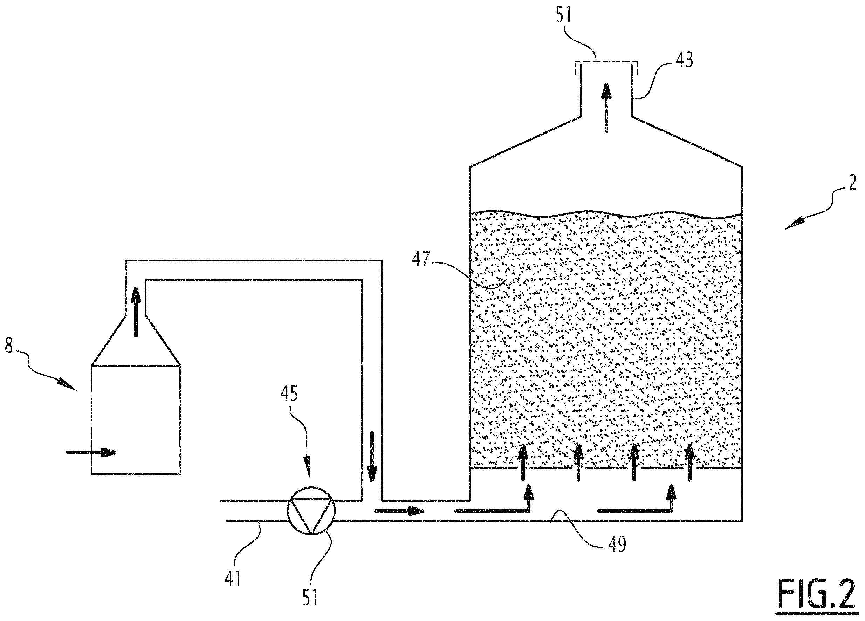

Un second mode de réalisation de l'ensemble de stockage de l'invention va maintenant être décrit, en référence à la figure 2. Seuls les points par lesquels le second mode de réalisation diffère du premier seront détaillés ci-dessous. Les éléments identiques ou assurant les mêmes fonctions seront désignés par les mêmes références dans les deux modes de réalisation.

Dans le second mode de réalisation, le local 2 est un silo à grains ayant une entrée d'air 41 communiquant avec l'extérieur et une sortie d'air 43 communiquant avec l'extérieur.

Le silo comprend une ventilation forcée 45 prévue pour assurer une circulation d'air de l'entrée d'air 41 à la sortie d'air 43 à travers les grains 47.

La ventilation forcée 45 est prévue pour assurer le séchage et/ou le refroidissement des grains 47.

Un collecteur 49 de distribution d'air est ménagé dans le silo 2, sous les grains. La ventilation forcée 45 comprend un organe 51 de circulation d'air tel qu'un ventilateur, dont l'aspiration est raccordée à l'entrée d'air 41 et le refoulement au collecteur 49.

La sortie d'air 43 est typiquement située en partie supérieure du silo.

En fonctionnement normal, l'organe de circulation 51 aspire l'air extérieur, le refoule dans le collecteur 49, l'air circulant jusqu'à la sortie d'air 43 à travers les grains 47 à partir du collecteur 49.

La ventilation forcée 45 est susceptible d'être mise à l'arrêt pendant l'injection du ou de chaque produit biocide et/ou phytoprotecteur.

Le dispositif 8 d'évaporation est placé à l'extérieur du silo 2. Il est agencé de manière à aspirer l'air extérieur et à refouler l'air chargé en vapeurs de produit depuis une zone proche de l'entrée d'air 41 vers la sortie d'air 43.

Par exemple, l'injection est faite en bas de la chambre recevant les grains 47, dans le collecteur 49.

L'injection est faite à un faible débit d'air.

Par exemple, le flux d'air circulant dans le garnissage 11 a un débit compris entre 1 et 6 m³ par heure et par 100 m³ de volume interne du local, de préférence entre 2 et 4 par heure et par 100 m³ de volume interne du local.

Le débit de liquide dans le garnissage 11 est typiquement compris entre 10 et 20 ml/m³ d'air.

L'air injecté a une concentration en vapeur du ou de chaque produit biocide et/ou phytoprotecteur supérieure à 50% de la concentration de saturation de la vapeur dudit produit dans l'air, de préférence supérieure à 80% de la saturation, encore de préférence supérieure à 90% de la saturation.

La vapeur du ou de chaque produit est plus lourde que l'air et a tendance à s'accumuler en bas du silo.

La vapeur est poussée vers la sortie d'air 43 par le flux d'air arrivant en continu du dispositif d'évaporation 8. Après une période de remplissage du silo par la vapeur du ou de chaque produit, la concentration en vapeur est maintenue dans le silo au-dessus de la limite souhaitée, ce qui permet un contact continu entre la vapeur et les grains. Ce contact prolongé conduit à l'effet biocide ou phytoprotecteur recherché.

De préférence, un filet 51 est placé sur chaque sortie d'air du silo, afin d'éviter que, suite au traitement des grains, les insectes présents puissent s'échapper et contaminer d'autres parties de l'installation,. Ces filets sont choisis pour laisser passer l'air mais bloquer les insectes.

Le procédé de traitement correspondant au second mode de réalisation de l'ensemble de stockage va maintenant être décrit. Seuls les points par lesquels le procédé diffère du celui pour le premier mode de réalisation de l'ensemble de stockage seront détaillés ci-dessous. Les éléments identiques ou assurant les mêmes fonctions seront désignés par les mêmes références.

Dans ce procédé, la ventilation forcée 45 du silo est à l'arrêt pendant l'injection du ou de chaque produit biocide et/ou phytoprotecteur.

Le flux d'air circulant dans le garnissage 11 du dispositif d'injection 8 a un débit compris entre 1 et 6 m³ par heure et par 100 m³ de volume interne du local.

La stratégie de traitement est du type dans laquelle l'étape de traitement soit comprend une phase d'injection continue unique, soit comprend plusieurs phases d'injection continues séparées par des phases d'attente sans injection de produit biocide et/ou phytoprotecteur. Dans les deux cas, chaque phase d'injection continue a une durée d'injection DI supérieure à 12 heures.

Typiquement, chaque phase d'injection dure entre un et 15 jours, de préférence entre deux et 5 jours.

Selon une variante applicable à tous les modes de réalisation, le liquide comprend plusieurs produits biocides et/ou phytoprotecteurs moyennement volatiles, ayant des pressions de vapeur respectives différentes les unes des autres à la température ambiante.

Avantageusement, les pressions de vapeur respectives des produits biocides et/ou phytoprotecteurs volatiles à la température ambiante s'étalent sur un intervalle de pression entre 1,3.10⁻⁵ bar et 4.10⁻³ bar.

Puisque, la demanderesse a observé qu'il existe une plage de pression de vapeur optimum pour l'obtention du maximum d'activité biocide et/ou phytoprotecteur recherché, considère qu'une tension de vapeur des liquides idéaux doit se situer entre 0,01 et 3 mmHg (1,3.10⁻⁵ bar et 4.10⁻³ bar) à température ambiante. Si la pression de vapeur est trop faible, le produit s'évapore lentement et la concentration de vapeur dans l'atmosphère interne du local ne peut pas atteindre une valeur suffisante pour obtenir l'effet recherché. Si la pression de vapeur est trop importante, les pertes vers l'extérieur du local peuvent être élevées.

Le fait d'utiliser plusieurs produits ayant des pressions de vapeur étagées permet de rester dans la plage de pression de vapeur recherchée pour un large domaine de températures autour de la température ambiante et d'avoir un plus large spectre d'activité par rapport à l'activité de chaque produit pris individuellement.

Selon une variante applicable à tous les modes de réalisation, l'ensemble de traitement 8 comprend un chauffage 51 de l'atmosphère interne du local 2. Ce chauffage est configuré pour chauffer ladite atmosphère interne pendant l'injection de la vapeur de produit, à une température comprise entre la température normale du local 2 et la température normale plus 5°C.

La température normale est la température à laquelle se trouve le local 2 en l'absence de traitement.

La température est augmentée pendant les phases d'injection pour augmenter la concentration de saturation de la vapeur du ou de chaque produit. Il est ainsi possible d'augmenter la concentration de vapeur du ou de chaque produit, ce qui conduit à une efficacité plus grande du traitement. La température est ramenée à sa valeur normale en stoppant le chauffage pendant les phases d'attente, sans injection de vapeur.

## Revendications

1. Procédé de traitement par au moins un produit biocide et/ou phytoprotecteur moyennement volatile, de température d'ébullition comprise entre 150 et 280°C, le procédé comprenant une étape de traitement au cours de laquelle un liquide contenant un produit ou un mélange de produits biocides et/ou phytoprotecteurs est évaporé et injecté dans l'atmosphère interne d'un local (2), le liquide étant évaporé à une température inférieure à 50°C, la concentration en vapeur de produit dans l'atmosphère interne du local (2) étant maintenue supérieure à 10% d'une concentration de saturation de la vapeur dudit produit dans ladite atmosphère à ladite température pendant une durée de saturation supérieure à 12 heures,
dans lequel le local (2) présente un volume interne supérieur à 200 m³ ;
dans lequel le liquide est stocké dans une réserve (7) et est évaporé dans un dispositif d'évaporation (8) comprenant un évaporateur (10) à garnissage (11) par mise en contact avec un flux d'air dans ledit garnissage (11), le flux d'air contenant les vapeurs de produit étant injecté dans le local (2), ledit dispositif d'évaporation (8) comprenant en outre:
- un dispositif (12) d'injection d'un flux du liquide dans l'évaporateur à garnissage (10) ;
- un organe (13) de circulation du flux d'air dans l'évaporateur à garnissage (10), la réserve (7) étant un bac placé verticalement sous le garnissage (11), le dispositif d'injection (12) étant agencé pour injecter le liquide au-dessus du garnissage (11).

2. Procédé selon la revendication 1, dans lequel au moins un des produits est choisi dans la liste de produits phytoprotecteurs ou biocides suivantes :
huile essentielle ; terpène ; alcool à chaîne courte de C6 à C10 saturé ou insaturé, comme par exemple, l'octanol, le 2-éthylhexanol ; produit de synthèse volatile, comme par exemple, l'hexanal, le diméthylnaphtalène et le 3-décène-2-one ; les acides organiques liquides à point d'ébullition élevé, tels que l'acide pélargonique et l'acide parabénique ; les esters avec activité biocide comme par exemple l'isovalérianate d'isoamyle.

3. Procédé selon la revendication 1 ou 2, dans lequel le local contient des produits végétaux, par exemple des céréales, stockés pendant une durée de stockage (DS), normalement supérieure à un mois.

4. Procédé selon la revendication 3, dans lequel l'étape de traitement comprend une phase d'injection continue de durée supérieure à 50% de la durée de stockage (DS), de préférence supérieure à 75% de la durée de stockage (DS) et encore de préférence supérieure à 90% de la durée de stockage (DS), au cours de laquelle la concentration en vapeur de produit dans l'atmosphère est maintenue entre 10% et 50% de la saturation.

5. Procédé selon l'une quelconque des revendications 1 à 3, dans lequel l'étape de traitement comprend une seule phase d'injection continue ayant une durée d'injection (DI) de 12 à 240 heures, ou comprend plusieurs phases d'injection continues séparées par des phases d'attente sans injection de produit biocide et/ou phytoprotecteur, chaque phase d'injection continue ayant une durée d'injection (DI) entre 12 heures et 240 heures, la concentration en vapeur de produit dans l'atmosphère étant maintenue entre 50% et 100% de la saturation pendant la ou chaque phase d'injection.

6. Procédé selon l'une quelconque des revendications précédentes, dans lequel le local (2) est un silo à grains ayant une entrée d'air (41) communiquant avec l'extérieur et une sortie d'air (43) communiquant avec l'extérieur, le silo comprenant une ventilation forcée (45) prévue pour assurer une circulation d'air de l'entrée d'air (41) à la sortie d'air (43) à travers les grains (47), la ventilation forcée (45) étant à l'arrêt pendant l'injection du ou de chaque produit biocide et/ou phytoprotecteur.

7. Ensemble de traitement, l'ensemble (3) comprenant :
- une réserve (7) de liquide contenant au moins un produit ou un mélange de produits biocides et/ou phytoprotecteurs moyennement volatiles, de température d'ébullition comprise entre 150 et 280°C,
- un dispositif (8) d'évaporation du liquide stocké dans la réserve (7) et d'injection du liquide évaporé dans l'atmosphère interne d'un local (2), le dispositif d'évaporation (8) étant configuré pour évaporer le liquide à une température inférieure à 50°C,
- un contrôleur (9) contrôlant le dispositif d'évaporation (8), configuré pour que la concentration en vapeur du ou de chaque produit dans l'atmosphère interne du local (2) soit maintenue supérieure à 10% d'une concentration de saturation de la vapeur dudit produit dans ladite atmosphère pendant une durée de saturation supérieure à 12 heures ;
dans lequel le local (2) présente un volume interne supérieur à 200 m^{3;}
dans lequel le dispositif d'évaporation (8) comprend un évaporateur (10) à garnissage dans lequel le liquide est évaporé par mise en contact avec un flux d'air dans ledit garnissage (11), l'évaporateur (10) étant configuré pour injecter le flux d'air contenant les vapeurs de produit dans le local (2), ledit dispositif d'évaporation (8) comprenant en outre:
- un dispositif (12) d'injection d'un flux du liquide dans l'évaporateur à garnissage (10) ;
- un organe (13) de circulation du flux d'air dans l'évaporateur à garnissage (10) ; la réserve (7) étant un bac placé verticalement sous le garnissage (11), le dispositif d'injection (12) étant agencé pour injecter le liquide au-dessus du garnissage (11).

8. Ensemble de traitement selon la revendication 7, dans lequel au moins un des produits est choisi dans la liste de produits phytoprotecteurs ou biocides suivante :
huile essentielle ; terpène ; alcool à chaîne courte de C6 à C10 saturé ou insaturé, comme par exemple, l'octanol, le 2-éthylhexanol ; produit de synthèse volatile, comme par exemple le l'hexanal, le diméthylnaphtalène et le 3-décène-2-one ; les acides organiques liquides à point d'ébullition élevé, tels que l'acide pélargonique et l'acide parabénique, les esters avec activité biocide comme par exemple l'isovalérianate d'isoamyle.

9. Ensemble de traitement selon la revendication 7 ou 8, dans lequel le local (2) contient des produits végétaux, par exemple des pommes de terre, stockés pendant une durée de stockage (DS) supérieure à un mois, le contrôleur (9) étant configuré pour réaliser une phase d'injection continue ayant une durée d'injection (DI) supérieure à 50% de la durée de stockage (DS), de préférence supérieure à 75% de la durée de stockage (DS) et encore de préférence supérieure à 90% de la durée de stockage (DS), au cours de laquelle la concentration en vapeur de produit dans l'atmosphère est maintenue entre 10% et 50% de la saturation.

10. Ensemble de traitement selon la revendication 7 ou 8, dans lequel le local (2) contient des produits végétaux, par exemple des céréales, le contrôleur (9) étant configuré pour réaliser une seule phase d'injection continue ayant une durée d'injection (DI) de 12 à 240 heures, ou plusieurs phases d'injection continues séparées par des phases d'attente sans injection de produit biocide et/ou phytoprotecteur, chaque phase d'injection continue ayant une durée d'injection (DI) de 12 à 240 heures, la concentration en vapeur de produit dans l'atmosphère étant maintenue entre 50% et 100% de la saturation pendant la ou chaque phase d'injection.

11. Ensemble de stockage de produits végétaux, l'ensemble de stockage (1) comprenant :
- un local (2), contenant de préférence des produits végétaux ; et
- un ensemble de traitement (3) selon l'une quelconque des revendications 7 à 10, configuré pour injecter le liquide évaporé dans l'atmosphère interne du local (2).

12. Ensemble de stockage selon la revendication 11, dans lequel le local (2) présente un volume interne supérieur à 200 m³.

## Patentansprüche

1. Verfahren zur Behandlung mit mindestens einem mäßig flüchtigen bioziden und/oder phytoprotektiven Produkt, mit einer Siedetemperatur zwischen 150 und 280°C, wobei das Verfahren einen Behandlungsschritt umfasst, in dessen Verlauf eine Flüssigkeit, die ein biozides und/oder phytoprotektives Produkt oder eine Mischung von bioziden und/oder phytoprotektiven Produkten enthält, verdampft und in die interne Atmosphäre eines Raums (2) eingespritzt wird, wobei die Flüssigkeit bei einer Temperatur von weniger als 50°C verdampft wird, wobei die Konzentration als Dampf des Produkts in der internen Atmosphäre des Raums (2) größer als 10% einer Sättigungskonzentration des Dampfes des Produkts in der Atmosphäre bei der Temperatur während einer Sättigungsdauer von mehr als 12 Stunden gehalten wird,
in dem der Raum (2) ein internes Volumen größer als 200 m³ aufweist;
in dem die Flüssigkeit in einem Vorrat (7) gespeichert wird und in einer Verdampfungsvorrichtung (8) verdampft wird, die einen Verdampfer (10) mit Packung (11) durch Inkontaktbringen mit einem Luftstrom in der Packung (11) umfasst, wobei der Luftstrom, der die Dämpfe des Produkts enthält, in den Raum (2) eingespritzt wird, wobei die Verdampfungsvorrichtung (8) ferner umfasst:
- eine Vorrichtung (12) zur Einspritzung eines Stroms der Flüssigkeit in den Verdampfer mit Packung (10);
- ein Organ (13) zur Zirkulation des Luftstroms in dem Verdampfer mit Packung (10), wobei der Vorrat (7) eine Wanne ist, die vertikal unter der Packung (11) angeordnet ist, wobei die Einspritzvorrichtung (12) angeordnet ist, um die Flüssigkeit oberhalb der Packung (11) einzuspritzen.

2. Verfahren gemäß Anspruch 1, in dem mindestens eines der Produkte aus der folgenden Liste von phytoprotektiven oder bioziden Produkten ausgewählt ist:
ätherisches Öl; Terpen; kurzkettiger Alkohol von C6 bis C10, gesättigt oder ungesättigt, wie zum Beispiel Octanol, 2-Ethylhexanol; flüchtiges Syntheseprodukt, wie zum Beispiel Hexanal, Dimethylnaphthalin und 3-Decen-2-on; die flüssigen organischen Säuren mit hohem Siedepunkt, wie Pelargonsäure und Parabensäure; die Ester mit biozider Aktivität wie zum Beispiel Isoamylisovalerianat.

3. Verfahren gemäß Anspruch 1 oder 2, in dem der Raum pflanzliche Produkte enthält, beispielsweise Getreide, die während einer Lagerdauer (DS), normalerweise größer als ein Monat, gespeichert werden.

4. Verfahren gemäß Anspruch 3, in dem der Behandlungsschritt eine Phase der kontinuierlichen Einspritzung mit einer Dauer von mehr als 50% der Lagerdauer (DS), vorzugsweise größer als 75% der Lagerdauer (DS) und noch bevorzugter größer als 90% der Lagerdauer (DS), umfasst, in deren Verlauf die Konzentration als Dampf des Produkts in der Atmosphäre zwischen 10% und 50% der Sättigung gehalten wird.

5. Verfahren gemäß einem beliebigen der Ansprüche 1 bis 3, in dem der Behandlungsschritt eine einzige Phase der kontinuierlichen Einspritzung mit einer Einspritzdauer (DI) von 12 bis 240 Stunden umfasst, oder mehrere kontinuierliche Einspritzphasen umfasst, die durch Wartephasen ohne Einspritzung von biozidem und/oder phytoprotektivem Produkt getrennt sind, wobei jede kontinuierliche Einspritzphase eine Einspritzdauer (DI) zwischen 12 Stunden und 240 Stunden aufweist, wobei die Konzentration als Dampf des Produkts in der Atmosphäre während der oder jeder Einspritzphase zwischen 50% und 100% der Sättigung gehalten wird.

6. Verfahren gemäß einem beliebigen der vorhergehenden Ansprüche, in dem der Raum (2) ein Getreidesilo mit einem Lufteinlass (41), der mit dem Äußeren kommuniziert, und einem Luftauslass (43), der mit dem Äußeren kommuniziert, ist, wobei das Silo eine Zwangsbelüftung (45) umfasst, die vorgesehen ist, um eine Luftzirkulation von dem Lufteinlass (41) zu dem Luftauslass (43) durch die Körner (47) hindurch sicherzustellen, wobei die Zwangsbelüftung (45) während der Einspritzung des oder jedes bioziden und/oder phytoprotektiven Produkts angehalten ist.

7. Behandlungsbaugruppe, wobei die Baugruppe (3) umfasst:
- einen Vorrat (7) an Flüssigkeit, die mindestens ein mäßig flüchtiges biozides und/oder phytoprotektives Produkt oder eine Mischung von mäßig flüchtigen bioziden und/oder phytoprotektiven Produkten enthält, mit einer Siedetemperatur zwischen 150 und 280°C,
- eine Vorrichtung (8) zur Verdampfung der in dem Vorrat (7) gespeicherten Flüssigkeit und zur Einspritzung der verdampften Flüssigkeit in die interne Atmosphäre eines Raums (2), wobei die Verdampfungsvorrichtung (8) ausgelegt ist, um die Flüssigkeit bei einer Temperatur von weniger als 50°C zu verdampfen,
- eine Steuerung (9), die die Verdampfungsvorrichtung (8) steuert, ausgelegt dafür, dass die Konzentration als Dampf des oder jedes Produkts in der internen Atmosphäre des Raums (2) größer als 10% einer Sättigungskonzentration des Dampfes des Produkts in der Atmosphäre während einer Sättigungsdauer von mehr als 12 Stunden gehalten wird;
in dem der Raum (2) ein internes Volumen größer als 200 m³ aufweist ;
in dem die Verdampfungsvorrichtung (8) einen Verdampfer (10) mit Packung umfasst, in dem die Flüssigkeit durch Inkontaktbringen mit einem Luftstrom in der Packung (11) verdampft wird, wobei der Verdampfer (10) ausgelegt ist, um den Luftstrom, der die Dämpfe des Produkts enthält, in den Raum (2) einzuspritzen, wobei die Verdampfungsvorrichtung (8) ferner umfasst:
- eine Vorrichtung (12) zur Einspritzung eines Stroms der Flüssigkeit in den Verdampfer mit Packung (10);
- ein Organ (13) zur Zirkulation des Luftstroms in dem Verdampfer mit Packung (10); wobei der Vorrat (7) eine Wanne ist, die vertikal unter der Packung (11) angeordnet ist, wobei die Einspritzvorrichtung (12) angeordnet ist, um die Flüssigkeit oberhalb der Packung (11) einzuspritzen.

8. Behandlungsbaugruppe gemäß Anspruch 7, in dem mindestens eines der Produkte aus der folgenden Liste von phytoprotektiven oder bioziden Produkten ausgewählt ist:
ätherisches Öl; Terpen; kurzkettiger Alkohol von C6 bis C10, gesättigt oder ungesättigt, wie zum Beispiel Octanol, 2-Ethylhexanol; flüchtiges Syntheseprodukt, wie zum Beispiel das Hexanal, Dimethylnaphthalin und 3-Decen-2-on; die flüssigen organischen Säuren mit hohem Siedepunkt, wie Pelargonsäure und Parabensäure, die Ester mit biozider Aktivität wie zum Beispiel Isoamylisovalerianat.

9. Behandlungsbaugruppe gemäß Anspruch 7 oder 8, in dem der Raum (2) pflanzliche Produkte enthält, beispielsweise Kartoffeln, die während einer Lagerdauer (DS) größer als ein Monat gespeichert werden, wobei die Steuerung (9) ausgelegt ist, um eine Phase der kontinuierlichen Einspritzung mit einer Einspritzdauer (DI) größer als 50% der Lagerdauer (DS), vorzugsweise größer als 75% der Lagerdauer (DS) und noch bevorzugter größer als 90% der Lagerdauer (DS), durchzuführen, in deren Verlauf die Konzentration als Dampf des Produkts in der Atmosphäre zwischen 10% und 50% der Sättigung gehalten wird.

10. Behandlungsbaugruppe gemäß Anspruch 7 oder 8, in dem der Raum (2) pflanzliche Produkte enthält, beispielsweise Getreide, wobei die Steuerung (9) ausgelegt ist, um eine einzige Phase der kontinuierlichen Einspritzung mit einer Einspritzdauer (DI) von 12 bis 240 Stunden, oder mehrere kontinuierliche Einspritzphasen, die durch Wartephasen ohne Einspritzung von biozidem und/oder phytoprotektivem Produkt getrennt sind, durchzuführen, wobei jede kontinuierliche Einspritzphase eine Einspritzdauer (DI) von 12 bis 240 Stunden aufweist, wobei die Konzentration als Dampf des Produkts in der Atmosphäre während der oder jeder Einspritzphase zwischen 50% und 100% der Sättigung gehalten wird.

11. Baugruppe zur Speicherung von pflanzlichen Produkten, wobei die Speicherbaugruppe (1) umfasst:
- einen Raum (2), der vorzugsweise pflanzliche Produkte enthält; und
- eine Behandlungsbaugruppe (3) gemäß einem beliebigen der Ansprüche 7 bis 10, ausgelegt, um die verdampfte Flüssigkeit in die interne Atmosphäre des Raums (2) einzuspritzen.

12. Speicherbaugruppe gemäß Anspruch 11, in dem der Raum (2) ein internes Volumen größer als 200 m³ aufweist.

## Claims

1. Method of treatment by at least one moderately volatile biocidal and/or phytoprotective product, having a boiling temperature comprised between 150 and 280°C, the method comprising a treatment step during which a liquid containing a biocidal and/or phytoprotective product or mixture of products is evaporated and injected into the internal atmosphere of a room (2), the liquid being evaporated at a temperature lower than 50°C, the concentration in vapor of product in the internal atmosphere of the room (2) being maintained greater than 10% of a saturation concentration of the vapor of said product in said atmosphere at said temperature for a saturation duration greater than 12 hours,
in which the room (2) has an internal volume greater than 200 m³ ;
in which the liquid is stored in a reserve (7) and is evaporated in an evaporation device (8) comprising a packed evaporator (10) (11) by bringing into contact with a flow of air in said packing (11), the flow of air containing the vapors of product being injected into the room (2), said evaporation device (8) further comprising:
- a device (12) for injecting a flow of the liquid into the packed evaporator (10);
- a member (13) for circulating the flow of air in the packed evaporator (10), the reserve (7) being a tank placed vertically under the packing (11), the injection device (12) being arranged to inject the liquid above the packing (11).

2. Method according to claim 1, in which at least one of the products is selected from the following list of phytoprotective or biocidal products :
essential oil; terpene; short-chain alcohol from C6 to C10 saturated or unsaturated, such as for example, octanol, 2-ethylhexanol; volatile synthetic product, such as for example, hexanal, dimethylnaphthalene and 3-decene-2-one; liquid organic acids with a high boiling point, such as pelargonic acid and parabenic acid; esters with biocidal activity such as for example isoamyl isovalerate.

3. Method according to claim 1 or 2, in which the room contains plant products, for example cereals, stored for a storage duration (DS), normally greater than one month.

4. Method according to claim 3, in which the treatment step comprises a continuous injection phase of duration greater than 50% of the storage duration (DS), preferably greater than 75% of the storage duration (DS) and still preferably greater than 90% of the storage duration (DS), during which the concentration in vapor of product in the atmosphere is maintained between 10% and 50% of the saturation.

5. Method according to any one of claims 1 to 3, in which the treatment step comprises a single continuous injection phase having an injection duration (DI) from 12 to 240 hours, or comprises several continuous injection phases separated by waiting phases without injection of biocidal and/or phytoprotective product, each continuous injection phase having an injection duration (DI) between 12 hours and 240 hours, the concentration in vapor of product in the atmosphere being maintained between 50% and 100% of the saturation during the or each injection phase.

6. Method according to any one of the preceding claims, in which the room (2) is a grain silo having an air inlet (41) communicating with the outside and an air outlet (43) communicating with the outside, the silo comprising a forced ventilation (45) provided to ensure a circulation of air from the air inlet (41) to the air outlet (43) through the grains (47), the forced ventilation (45) being stopped during the injection of the or of each biocidal and/or phytoprotective product.

7. Treatment set, the set (3) comprising:
- a reserve (7) of liquid containing at least one moderately volatile biocidal and/or phytoprotective product or mixture of products, having a boiling temperature comprised between 150 and 280°C,
- a device (8) for evaporation of the liquid stored in the reserve (7) and for injection of the evaporated liquid into the internal atmosphere of a room (2), the evaporation device (8) being configured to evaporate the liquid at a temperature lower than 50°C,
- a controller (9) controlling the evaporation device (8), configured so that the concentration in vapor of the or of each product in the internal atmosphere of the room (2) is maintained greater than 10% of a saturation concentration of the vapor of said product in said atmosphere for a saturation duration greater than 12 hours;
in which the room (2) has an internal volume greater than 200 m³;
in which the evaporation device (8) comprises a packed evaporator (10) in which the liquid is evaporated by bringing into contact with a flow of air in said packing (11), the evaporator (10) being configured to inject the flow of air containing the vapors of product into the room (2), said evaporation device (8) further comprising:
- a device (12) for injecting a flow of the liquid into the packed evaporator (10);
- a member (13) for circulating the flow of air in the packed evaporator (10); the reserve (7) being a tank placed vertically under the packing (11), the injection device (12) being arranged to inject the liquid above the packing (11).

8. Treatment set according to claim 7, in which at least one of the products is selected from the following list of phytoprotective or biocidal products:
essential oil; terpene; short-chain alcohol from C6 to C10 saturated or unsaturated, such as for example, octanol, 2-ethylhexanol; volatile synthetic product, such as for example the hexanal, dimethylnaphthalene and 3-decene-2-one; liquid organic acids with a high boiling point, such as pelargonic acid and parabenic acid, esters with biocidal activity such as for example isoamyl isovalerate.

9. Treatment set according to claim 7 or 8, in which the room (2) contains plant products, for example potatoes, stored for a storage duration (DS) greater than one month, the controller (9) being configured to carry out a continuous injection phase having an injection duration (DI) greater than 50% of the storage duration (DS), preferably greater than 75% of the storage duration (DS) and still preferably greater than 90% of the storage duration (DS), during which the concentration in vapor of product in the atmosphere is maintained between 10% and 50% of the saturation.

10. Treatment set according to claim 7 or 8, in which the room (2) contains plant products, for example cereals, the controller (9) being configured to carry out a single continuous injection phase having an injection duration (DI) from 12 to 240 hours, or several continuous injection phases separated by waiting phases without injection of biocidal and/or phytoprotective product, each continuous injection phase having an injection duration (DI) from 12 to 240 hours, the concentration in vapor of product in the atmosphere being maintained between 50% and 100% of the saturation during the or each injection phase.

11. Set for storage of plant products, the storage set (1) comprising:
- a room (2), preferably containing plant products; and
- a treatment set (3) according to any one of claims 7 to 10, configured to inject the evaporated liquid into the internal atmosphere of the room (2).

12. Storage set according to claim 11, in which the room (2) has an internal volume greater than 200 m³.
